Europäisches Patentamt

European Patent Office

Office européen des brevets

(19)

(11) Publication number: **0 253 772**
**A2**

(12) # EUROPEAN PATENT APPLICATION

(21) Application number: **87810391.0**

(22) Date of filing: **09.07.87**

(51) Int. Cl.⁴: **A 61 K 7/30**

(30) Priority: **15.07.86 US 885976    30.04.87 US 45103**

(43) Date of publication of application:
**20.01.88 Bulletin 88/03**

(84) Designated Contracting States:
**AT BE CH DE FR GB IT LI LU NL SE**

(71) Applicant: **WARNER-LAMBERT COMPANY**
**201 Tabor Road**
**Morris Plains New Jersey 07950 (US)**

(72) Inventor: **Eoga, Anthony B. J.**
**321 Rexland Drive**
**Boonton, N.J. 07005 (US)**

(74) Representative: **Silbiger, Jakob, Dr.**
**c/o CAPSUGEL AG Münchensteinerstrasse 41**
**CH-4002 Basel (CH)**

(54) Denture cleansing and/or washing compositions containing a bleach activator.

(57) Compact, cohesive, hard bleach activator granules, particularly well suited in the formulation of cleansing and/or washing compositions, said bleach activator granules comprising bleach activator, at least one perborate salt and a polymeric fluorocarbon binder inert to the bleach activator. Effervescent cleansing compositions, preferably in tablet form, containing the herein improved bleach activator granules, are also disclosed.

An improved effervescent cleansing composition is disclosed. the composition comprises a monopersulfate salt, a bleach activator, at least one perborate salt and a polymeric fluorocarbon binder. The polymeric fluorocarbon binder and at least a portion of the perborate salts are present in a compacted granulated mixture.

EP 0 253 772 A2

Bundesdruckerei Berlin

# 0 253 772

**Description**

## DENTURE CLEANSING AND/OR WASHING COMPOSITIONS CONTAINING A BLEACH ACTIVATOR

The present invention relates generally to cleansing compositions and more particularly to cleansing compositions in tablet form useful for denture cleansing and washing.

Cleanser compositions, particularly those compositions having utility for hard surface cleaning applications, utilize oxidizing agents and bleaching agents in concert to remove visible stains, while at the same time providing the capability for the removal of scale or plaque buildup on the surfaces being cleaned. Thus, a variety of cleansing compositions are known and are prepared with abrasive materials for use as scouring cleansers or without abrasives for purposes of mild surface cleaning applications, such as passive dispersion in a liquid medium, e.g., water, for soaking applications, such as for the cleaning of dentures. All of these compositions employ a variety of sulfate salts, such as bisulfates, monopersulfates and sulfates as detergents, oxidizers and the like and also utilize alkali metal and alkaline earth metal halides as bleaches. Such compositions generally also included perborate, carbonate and phosphate salts in varying amounts to provide effervescence and activation. Representative cleansing compositions covering these various applications are set forth in U.S. Patent No. 3,337,466 to Puetzer et al., U.S. Patent No. 3,704,227 to Hill, U.S. Patent No. 4,362,639 to Eoga, and U.S. Patent No. 4,409,118 to Eoga, all of which are incorporated herein by reference.

In the instances wherein the above-mentioned cleansing compositions contain one or more perborate salts and the compostions are prepared into tablets by compression, the formulation of the compositions has presented certain hardships in that they are difficult to compress and the resulting tablets lack mechanical strength. These problems are due primarily to physical properties of the perborate salts which are employed. In particular, anhydrous sodium perborate is commercially available as a fluffy powder having a low specific weight and density and therefore resistant to compaction and agglomeration. To a much lesser extent, this same difficulty is experienced with another perborate salt additive, sodium perborate monohydrate.

Prior art attempts to remedy these deficiencies have taken varied approaches. For example, some prior art focuses on the addition of greater amounts of standard tableting aids such as talc, sodium benzoate and the like. The addition of greater amounts of these ingredients, however, while remedying the difficulties of initial processing and tablet formation, carry with them certain other drawbacks, namely that the formed tablets exhibit retarded action in use that renders them less commercially desirable. In particular, the increased amounts of tableting aids tend to prolong the disintegration time of the tablet with the result that the activity of the tablet is delayed and in some instances slightly suppressed and therefore less attractive to potential consumers.

U.S. Patent No. 4,115,519 to Brichard et al. discloses a process for the manufacture of sodium perborate monohydrate that purportedly results in the preparation of granules of the monohydrate possessing the desired particle size, specific weight, abrasion resistance and flowability sought for use in connection with the composition of dental cleanser tablets. This technique is complex and costly and requires specialized apparatus to conduct a fluidized bed particle formation in contact with hydrogen peroxide.

U.S. Patent No. 3,340,152 to Hotko discloses that polyfluorocarbons may be utilized in the manufacture of tablets, as lubricants, and in amounts ranging from about 1% to about 15% by weight to supplant such known lubricants as magnesium stearate, sodium lauryl sulfate, polyethylene-glycols and the like. The patentee suggests that the fluoropolymer may be added directly to the tableting mixture in its capacity and amount as a lubricant and purportedly has a favorable effect on the tablet-forming process. There is no disclosure in Hotko that the fluoropolymers would serve as agglomeration or compressible composition aids to facilitate the preparation of granulated materials of increased and improved specific weight.

In U.S. Patent No. 4,362,639 efforts have been devoted to creating a cleanser of improved after odor and tarnish resistance properties. The cleanser utilizes as critical ingredients an oxidizing agent such as monopersulfate salt; a halide bleaching promoter, such as an alkali metal or alkaline earth metal; an effervescent compound, such as a perborate salt; and an ammonium ion contributor to inhibit the evolution of chlorine-like odor and taste.

U.S. Patent No. 4,405,486 discloses a method for preparing granulated perborate salts having improved utility in compressible compositions such as those formed into denture cleanser tablets. The method comprises forming a mixture of one or more perborate salts with a polymeric fluorocarbon. The particles prepared exhibit a combination of improved hardness and specific weight that facilitates their participation in the compressible cleanser composition, together with enhanced disintegration characteristics that are favorably imparted to the cleanser tablets.

Additionally, U.S. Patent No. 4,409,118 discloses forming a tablet which has good mechanical strength and excellent dissolution speed and cleansing efficiency. The addition of a polymeric fluorocarbon to a pre-formed compacted granulated mixture of perborate salt allows for the improved composition and dimensional stability without the tendency to stick to tablet forming equipment.

In turn, U.S. Patent No. 4,518,520 discloses the preparation of a cleansing composition that is particularly suited for compression into tablet form and which results in, upon dissolution in water, improved clarity of solution and improved speed of dissolution. More particularly, these cleansing compositions comprise a phosphate salt mixture, an improved perborate salt mixture, comprising a combination of anhydrous perborate

2

and monohydrate perborate and wherein said combination contains a portion of polymeric fluorocarbon.

Denture cleansers and washing compositions which contain bleach activators are also known. See, for example, U.S. Patent No. 4,422,950. These bleach activators usually comprise carboxylic acid derivatives which in aqueous bleach solutions react with the percompounds, e.g., perborate, with the formation of peroxyacids and therefore increase the bleaching action of the mixtures even at low washing temperatures due to their contribution to increase the activated oxygen. In order to improve the storage of such bleach activation containing compositions, the bleach activator is generally used in the form of granulated particles which require the use of a binder material. These binders, however, tend to prevent the granule from dissolving rapidly when placed into the cleaning medium and in some cases tend to sediment in the cleaning medium where they remain substantially inactive during the washing or cleansing operation. For bleach activators, the unreactive bleach activator can be as high as 50% or more. The result is a reduced peroxy acid yield and consequently a reduced bleach efficiency. Thus the type of binding material is significant to the amount of peroxy yield. Most binders require water as a means of applying the binder material. Any amount of water would cause premature decomposition of the percompounds and also affects the long term storage stability of the granules.

Accordingly, it has been surprisingly discovered herein that improved cleanser and washing compositions can be provided by incorporating therein improved bleach activator granules. The improved bleach activator granules of the present invention for use in washing and/or cleansing compositions comprise:

(i) a bleach activator;

(ii) An anhydrous alkali metal perborate; and/or

(iii) a perborate monohydrate of a metal selected from the group consisting of an alkali metal, an alkaline earth metal and mixtures thereof; and

(iv) a polymeric fluorocarbon binder.

Furthermore, improved effervescent cleansing and/or washing compositions, in tablet or powder form are provided herein by incorporating the improved bleach activator granules therein. A typcial cleansing and/or washing composition comprises:

(i) from about 20 to about 60% by weight, of the final composition, of a monopersulfate salt;

(ii) a bleach activator;

(iii) an anhydrous alkali metal perborate; and/or

(iv) a perborate monohydrate of a metal selected from the group consisting of an alkali metal, an alkaline earth metal and mixtures thereof; and

(v) a polymeric fluorocarbon binder which is inert to the bleach activator, wherein said bleach activator and at least a portion of said anhydrous perborate and/or perborate monohydrate and said polymeric fluorocarbon binder are present in a compacted granule mixture.

The improved bleach activators of the present invention comprise a bleach activator compound which reacts with a percompound forming a peroxyacid. Typical bleach activators are carboxylic anhydrides, carboxylic acid esters and N-acyl or O-acyl substituted amides or amines. Such bleach activators are described for example in a serious of articles by Allen H. Gilbert in Detergent Age, June 1967 pages 18-20, July 1967 pages 30-33, and August 1967 pages 26,27, and 67 the entire contents of which are incorporated by reference herein. A representative but by no means comprehensive list of activators which can be used in the present invention is as follows:

(a) N-diacylated and N,N'-tetraacylated amines, such as N,N,N',N'-tetraacetylmethylenediamine or -ethylenediamine, N,N-diacetylaniline and N,N-diacetyl-p-toluidine or 1,3-diacylated hydantoins, as for example, the compounds 1,3-diacetyl-5,5-dimethylhydantoin and 1,3-dipropionylhydantoin;

(b) N-alkyl-N-sulphonyl-carbonamides, for example, the compounds N-methyl-N-mesyl-acetamide, N-methyl-N-mesylbenzamide, N-methyl-N-mesyl-p-nitrobenzamide, and N-methyl-N-mesyl-p-methoxybenzamide;

(c) N-acylated cyclic hydrazides, acylated triazoles or urazoles, for example monacetylmaleic acid hydrazide;

(d) O,N,N-trisubstitued hydroxylamines, such as O-benzoyl-N,N-succinylhydroxylamine, O-acetyl-N,N-succinyl-hydroxylamine, O-p-methyoxybenzoyl, N,N-succinyl-hydroxylamine, O-p-nitrobenzoyl N,N-succinyl-hydroxylamine and O,N,N-triacetyl-hydroxylamine;

(e) N,N'-diacyl-sulphurylamides, for example N,N'-dimethyl-N,N'-diacetylsulphurylamide and N,N'-diethyl-N,N'-dipropionyl-sulphurylamide;

(f) Triacyl cyanurates, for example triacetyl cyanrate and tribenzoyl cyanurate;

(g) Carboxylic acid anhydrides, such as benzoic anhydride, m-chlorobenzoic anhydride, phthalic anhydride, and 4-chlorophtalic anhydride;

(h) Sugar esters, for example glucose pentaacetate;

(i) 1,3-diacyl-4,5-diacyloxy-imidazolidines, for example, 1,3-diformyl-4,5-diacetoxy-imidazolidine, 1,3-diacetyl-4,5-diacetoxy-imidazolidine, 1,3-diacetyl-4,5-dipropionyloxy-imidazolidine;

(j) Tetraacetylglycoluril and tetrapropionylglycoluril;

(k) Diacylated 2,5-diketopiperazines, such as 1,4-diacetyl-2,5-diketopiperazine, 1,4-dipropionyl-2,5-diketopiperazine and 1,4-dipropionyl-3,6-dimethyl-2,5-diketopiperazine;

(l) Acylation products of propylenediurea and 2,2-dimethylpropylenediurea, especially the tetracetyl or tetrapropionyl propylenediurea and their dimethyl derivatives;

(m) Carbonic acid ester, for example the sodium salts of p-(ethoxycarbonyloxy)-benzoic acid and p-(propoxycarbonyloxy)-benzenesulphonic acid; and

(n) alpha-acyloxy-(N,N')polyacylmalonamides, such as alpha-acetoxy-(N,N')-diacetylmalonamide.

N,N,N',N'-tetraacetylethylenediamine (TEAD) is a preferred bleach activator in accordance with the present invention.

For purposes of the present invention, the amount of bleach activator compound utlized is generally from about 10 to about 50% of the granule, preferably 25-35% by weight of the total weight of the granule.

The perborate salts employed in the bleach activator granules of the present invention may be selected from alkali metal perborates and alkaline earth metal perborates and more particularly may be selected from alkali metal and/or alkaline earth metal perborate monohydrates and anhydrous alkali metal perborates. Thus, the sodium and potassium salts of the perborate monohydrate and anhydrous perborates may be utilized and preferably the perborate salts comprise sodium perborate monohydrate and anhydrous sodium perborate. Also included are the ammonium, calcium and magnesium salts of monohydrate and anhydrous perborates.

The anhydrous perborate salt is generally utilized in an amount of from about 5 to about 50 weight percent, preferably about 10 to 30 weight percent, based on the total weight of the bleach activator granule. In turn, the perborate monohydrate is employed in an amount of from about 30 to about 60 weight percent, preferably about 40 to about 50 weight percent, based on the total weight of the bleach activator granule.

The perborate salt component functions in a variety of capacities within the present compositions, as it provides cleaning action, as well as promoting the activity of the compositions by initiating effervescence as well as inhibiting tarnish and corrosion of susceptible substrates immersed in solutions of the present composition.

A further feature of the present improved bleach activator is the utilization of a binder material, namely a polymeric fluorocarbon. The polymeric fluorocarbon may be selected from a well known class of polymeric and copolymeric substances comprised of carbon and fluorine, which in addition may contain hydrogen and/or chlorine. The fluorocarbon may include at least one fluoroolefin, for example, polytetrafluoroethylene, copolymers of tetrafluoroethlyene and hexafluoropropylene and copolymers of vinylidene fluoride and hexafluoropropylene would be included. The preferred polymeric fluorocarbon comprises polytetrafluoroethylene.

In accordance with the present invention, the polymeric fluorocarbon binder utilized in the bleach activator granule is used in an amount in the range of from about 0.01 to about 1% by weight, preferably 0.05 to about 1% by weight, based on the total weight of the bleach activator granule. The fluorocarbon polymers may be utilized in the form of powders having particle sizes acceptable for combination with the perborate salts and bleach activator, and preferably ranging up to about 150 microns in size. The exact particle size may vary, and an average size of from about 25 to about 75 microns may be used. The exact particle size of the polymeric fluorocarbon is not critical to the practice of the present invention.

It has been found herein that the combination of a polymeric fluorocarbon, such as polytetrafluoroethylene, perborate salt mixture and bleach activator compound results in the formation of a compacted and cohesive hard granule which allows the bleach activator compound to dissolve rapidly in a clear solution when used with foaming detergent powders or granules or when used in a formulation such as a denture cleanser tablet or powder. The utilization of a polymeric fluorocarbon, such as polytetrafluoroethylene, provides strength to the granule which contains the perborate as an essential effervescent ingredient and is inert to the bleach activator and thus is easily dispersible in a washing medium.

Polytetrafluoroethylene is the preferred binder and compactor because the rate of dissolution of the granule is not appreciably reduced. Thus, the composition provides the bleach activator compounds with the available peroxy acid to improve the bleaching and cleansing. Furthermore, in aqueous medium, the preferred bleach activator granules herein provides a pH in the range of from about 10 to about 11.5.

In accordance with a further embodiment of the present invention, improved cleansing compositions, preferably in tablet form, comprise a monopersulfate salt, such as an alkali metal salt, typically potassium monopersulfate, in an amount of from about 20 to about 60 weight percent, preferably about 30 to about 50 weight percent of the total cleansing composition. The cleansing composition may also typically contain a phosphate salt in an amount of from about 5% to about 15% by weight of the total composition, including the bleach activator granules hereinbefore described. Preferably, the phosphate salt may be present in an amount ranging from about 8 to about 12% by weight and comprises alkali metal phosphates and/or alkaline earth metal phosphates, with alkali metal phosphates being preferred. More specifically, a preferred alkali metal phosphate comprises sodium tripolyphosphate.

The phosphate component serves as a cleanser. In the instances where the present compositions are utilized as denture cleanser, the phosphate component attacks and disintegrates dental plaque that forms on the surface of the dentures. This cleaning role is in addition to the function of the phosphates as builders.

In addition to the ingredients set forth above, the cleansing and/or washing compositions of the present invention may contain a variety of additional ingredients selected on the basis of desired end use. Thus, for example, the compositions may include detergent compounds, such as organic and inorganic detergents, including nonionic detergents such as the various polyoxyethylene ethers of aromatic and aliphatic alcohols, as well as the polyoxyethylene ethers of hydrophobic propylene oxide polymers. These compounds assist in maintaining a foaming action in aqueous solution.

An example of a preferred detergent is a sodium lauryl sulfoacetate such as that available under th product designation Lathanol LAL Powder. Suitable amounts of such a detergent are about 0.5% to about 0.9% by weight of the total composition with about 0.6% to about 0.8% by weight being preferred.

Also, the compositions may contain other adjuvant materials, that may be inorganic or organic in structure. Thus, inorganic water-soluble alkaline builders such as alkali and alkaline earth metal carbonates, hydroxides, and mixtures may be added. Particularly, sodium carbonate may be present in an amount of up to about 20% by weight, as it functions not only as a builder, but enhances effervescence and assists in stabilizing the pH of the solutions obtained from the composition.

The present compositions may also contain sequestrants or chelating agents for the purpose of maintaining solution clarity, in the instance where the compositions are placed in solution. The sequestrants or chelating agents may also assist in the inhibition of corrosion and tarnish of particles soaked in solutions containing the present compositions. Useful sequestrants include ethylene diamine tetracetic acid (EDTA) and its corresponding alkali salts, as well as other polyfunctional organic acids, such as citric acid, maleic acid and their corresponding salts. The sequestrants may be present in amounts of from about 2 to about 6% by weight, preferably 3 to about 5% by weight of the total composition.

In the instance where the composition is to be prepared for use as a denture cleanser, other additives such as flavorings, colorants, perfumes and the like may be added in various amounts as mentioned earlier. For example, the flavorings may include varieties of mint, oil of clove, artificial vanilla flavoring, and others. These materials may be included and blended in various combinations within the scope of the presnet invention. The choice of the required amounts is likewise within the skill of the art.

In the instance where the present cleansing compositions are formulated for use as denture cleansers, the colorants useful herein are those known as F.D.&C. and D.&C. dyes and lakes. These materials are certified by the Federal Food and Drug Administration as acceptable for use in food, drug and cosmetic applications, and drug and cosmetic colorings. The materials acceptable for the foregoing spectrum of use are preferably water soluble, and include indigoid dye, known as F.D.&C. blue No. 2, which is the disodium salt of 5,5'-indigotin-disulfonic acid. Similarly, the dye known as F.D.&C. Green No. 1, comprises a triphenylmethane dye and is the monosodium salt of 4-[4-(N-ethyl-p-sulfobenzylamino)-diphenylmethylene]-[1-(N-ethyl-N-p-sulfoniumbenzyl)-$\Delta^{2,5}$-cyclohexadienimine]. A full recitation of all F.D.&C. and D.&C. and their corresponding chemical structures may be found in the Kirk-Othmer Encyclopedia of Chemical Technology, 3rd Ed. at Volume 6, pages 561-595, which text is accordingly incorporated herein by reference. Dyes and colorants will fade at different rates and may be chosen to provide specific end points.

The foregoing colorants may be blended with each other in a variety of combinations. It is particularly desirable that the colorants be chosen so that the composition when initially dissolved will present a deep hue. This is important in the instance where the composition serves as a denture cleanser, as the fading phenomenon embodied in denture cleansers can be more easily observed by the end user.

The present invention further includes a method for the preparation of the bleach activator granules herein which comprises mixing anhydrous sodium perborate, sodium perborate monohydrate, bleach activator and polytetrafluoroethylene in the dry state and directly compacting an sizing by the use of an oscillating granulator or a fitzmill or any other sizing technique or method known in the art. The resulting granules generally have a particle size of from about 10 mesh to about 100 mesh, preferably from about 12 to 30 mesh.

In accordance with the present invention, the compositions may be prepared in tablet form without the need for increased addition of ingredients such as excipients, tableting agents and the like. While such ingredients may be added, the amounts of these ingredients may be reduced, due to th favorable effect exerted by the polymeric fluorocarbon present in the compacted granules of the perborate salt. Naturally, minor additional quantities of ingredients may be made for their stated purpose, such as for lubrication and the like, however such additions and their respective amounts are not critical and do not form a part of the present invention.

As stated above, improved cleanser and washing compositions can be provided by incorporating improved bleach activator granules therein. Although, it is preferred to use the described bleach activator granules, an efficacious denture cleansing and/or washing composition is produced by incorporating free bleach activator into the composition--i.e., the bleach activator is not first formed into a granulated mixture (granules) with the perborates, but rather is added as a separate ingredient to the composition. Such compositions provide yet another aspect of this invention and are described below. Those skilled in the art will appreciate that, unless stated otherwise, the above disclosure pertaining to the various aspects and components of the compositions comprising bleach activator granules also apply to the compositions described below which do not contain bleach activator granules.

Thus, in another aspect, this invention provides an improved effervescent cleansing and/or washing composition, in tablet or powder form, comprising a monopersulfate, anhydrous perborates and/or perborate monohydrates, a polymeric fluorocarbon, a bleach activator, and optionally, other known in the art additives used in denture cleansing compositions. Typically, there is provided an improved effervescent cleansing composition comprising:

(i) from about 20% to about 60% by weight of the total composition of a monopersulfate salt;

(ii) an effective amount of a bleach activator;

(iii) an effective amount of an anhydrous alkali metal perborate; and/or

(iv) an effective amount of a perborate mononhydrate of a metal selected from the group consisting of an alkali metal, an alkaline earth metal and mixtures thereof; and

(v) an effective amount of a polymeric fluorocarbon binder which is inert to the bleach activator;

wherein

at least a portion of said anhydrous perborate and/or perborate monohydrate and said polymeric fluorocarbon binder are present in a compacted granulated mixture.

Those skilled in the art will appreciate that the compositions of this invention can be used in powder form and in tablet form. The compositions ultimatly compacted into tablets will generally have an effective amount of a polymeric fluorocarbon binder in addition to the polymeric fluorocarbon binder which is present in the compacted granulated mixtures. In general, amounts of about 0.05% to about 0.2% by weight of the total composition are useful with about 0.09% to about 0.12% by weight being preferred. The compositions may optionally contain effective amounts of such other known in the art additives such as, phosphate salts; detergents; adjuvant materials such as inorganic water-soluble alkaline builders; chelating agents (chelants) or sequestrants; flavorings; colorants (dyes); perfumes; excipients; fillers, such as sodium sulfate; preservatives, such as sodium benzoate; effervescent material combinations such as citric acid with sodium bicarbonate and/or sodium carbonate; lubricants to aid tableting, such as magnesium stearate; and the like.

In general, a portion of the perborate salts are in admixture together with a polymeric fluorocarbon in the form of a compacted granulated mixture (granules). In addition, the composition also contains an effective amount of the perborate monohydrate present in the composition, but not present as part of the compacted granulated mixture--i.e. free of the compacted granulated mixture. In other words, an amount of the perborate monohydrate is added to the composition as a separate ingredient rather than as part of the compacted granulated mixtures and is therefore not bound within nor present within the granulated mixture. Usually, up to about 11% by weight of the total composition of the perborate monohydrate may be added as a component separate from the amount of perborate monohydrate which may be present in the compacted granulated mixture. Since too little amounts of the separately added perborate monohydrate may not prove effective, it is preferred that about 7% to about 11% by weight of the total composition be used.

The anhydrous perborate is usually present in the compacted granulated mixture in amounts of about 2% to about 8% by weight of the total composition with about 3% to about 5% by weight being preferred. The perborate monohydrate is usually present in the compacted granulated mixture in amounts of about 2% to about 8% by weight of the total composition with about 3% to about 8% being prefered. The ratio of anhydrous perborate to monohydrate perborate (perborate monohydrate) is usually within the range of about 1.5:1 to about 1:1.5. The compacted granulated mixture also contains about 0.4% to about 0.7% by weight of the compacted granulated mixture of a polymeric fluorocarbon. Suitably, the compacted granulated mixture is present in amounts of about 5% to about 10% by weight of the total composition with aobut 6% to about 9% by weight being preferred.

The bleach activator is usually present in amounts of about 5% to about 15% by weight of the total composition with about 5% to about 10% by weight being preferred, and about 6% to about 8% by weight being most preferred. Preferably, the bleach activator is N,N,N',N'-tetraacetylethylene diamine (TAED).

Those skilled in the art will appreciate that in any given composition the total percentages of the various components of the composition equals 100%.

A fuller understanding of the present invention will be gained from a review of the following illustrative examples. Unless specified otherwise, all amounts expressed as percent, are intended to be a percent by weight.

## EXAMPLE 1

This example illustrates the preparation of a bleach activator granule in accordance with the present invention.

In this example, 338 grams of sodium perborate monohydrate, 83 grams of anhydrous sodium perborate, 200 grams N,N,N',N',-tetraacetylethylenediamine (TAED) and 2 grams of polytetrafluoroethylene powder identified as Grade F5A (E.I. duPont DeNemours & Co., Inc.) are admixed in the dry state. Mixing is conducted for approximately 1-3 minutes, after which the mixture is compressed to a hard tablet with a hardness of over 30 SCU. (Strong Cobb Units). Thereafter, the granules are passed through an oscillating granulator having a 12 mesh size screen.

## EXAMPLE 2

The procedure followed in Example 1 is repeated, with the exception that 296 grams of sodium perborate monohydrate and 182 grams of anhydrous sodium perborate are used in combination with the polytetrafluoroethylene and TAED. The resulting granules were of comparable hardness to those prepared in Example 1.

## EXAMPLE 3

A denture cleansing composition is prepared incorporating the bleach activator granules prepared in accordance with Example 1. The specific ingredients of the denture cleansing composition are set forth in Table I, below.

## Table I

### Denture Cleansing Composition

| Ingredient | Amount (Grams)/per tablet |
| --- | --- |
| Sodium Bicarbonate USP Granular No. 5 | 0.3420 |
| Dyes | 0.0051 |
| Water | 0.0050 |
| Ethylenediamine Tetraacetic Acid, Tetrasodium Salt Dihydrate/Pure | 0.1200 |
| Sodium Tripolyphosphate Anhydrous Granular Technical Grade | 0.3180 |
| Sodium Carbonate (Soda Ash Dense) | 0.2850 |
| Citric Acid Anhydrous USP Fine Granular | 0.1190 |
| Sodium Sulfate Anhydrous | 0.1500 |
| Potassium Monopersulfate | 1.2210 |
| Mint Frangrance | 0.3000 |
| Surfactant | 0.0200 |
| Excipients | 0.0460 |
| Polytetrafluoroethylene Powder | 0.0028 |
| Bleach Activator Granules (Example 1) | 0.6230 |

The above denture cleansing composition, including the bleach activator granule of Example 1, is then mixed and pressed into tablets using a tablet press having a tablet die of 27/32″. Each tablet weighs about 3.55 grams and has a hardness of about 16 SCU on edge Heuberlein. The tablets are about 0.2 inches thick and upon testing are found to have available oxygen of 115 mg/tablet with a disintegration time of max. 180 seconds in 120 milliliters of 45°C water.

The performance of these tablets is considered above average in the measured parameters and the tablets containing the bleach activator granules of the present invention are determined to be desirable for use in denture cleanser compositions in tablet form.

EXAMPE 4

A denture cleansing composition of this invention was prepared having the formulation presented in Table II. In this formulation the bleach activator (TAED) was added to the composition without first being incorporated into a granule.

## Table II

___

### Denture Cleansing Composition

| Ingredient | Amount (mg/tablet) |
|---|---|
| **Pregranulation** | |
| Anhydrous Sodium Perborate | 141.88 |
| Sodium Perborate Monohydrate | 87.06 |
| Polytetrafluorethylene | 1.06 |
| Total Pregranulation | 230.00 |
| | |
| **Final Mixture** | |
| Sodium Carbonate(Soda Ash Dense) | 300.00 |
| Potassium Monopersulfate | 850.00 |
| Lathanol LAL Powder | 20.00 |
| Pregranulation | 230.00 |
| Sodium Tripolyphosphate Anhydrous | 335.00 |
| Sodium Bicarbonate USP Granular No. 5 | 360.00 |
| Dyes | 3.08 |
| Citric Acid Anhydrous USP Fine Granular | 125.00 |
| Sodium Perborate Monohydrate | 265.00 |
| Flavor | 30.00 |
| Sodium Benzoate | 48.00 |
| Sodium Sulfate | 160.00 |
| TAED | 200.00 |
| Magnesuim Stearate | 1.00 |
| Polytetrafluoroethylene | 3.00 |
| Total Final Mixture | 2,930.08 |

___

The pregranulation of Table II was produced in a manner similar to the production of the bleach activator granules of Example 1, except no bleach activator was added to the pregranulation. Thus, the ingredients of the pregranulation were mixed together for about 1 to about 3 minutes, the mixture was compacted (compressed) into tablets having a hardness of 30 SCU. Thereafter, the tablets were comminuted and the granules formed by the comminuation were passed through a 20 mesh size screen. Preparation of granulated perborate salts (pregranulation) is described in U.S. 4,405,486 issued Spetember 20, 1983, the disclosure of which is incorporated herein by reference thereto.

Although, there is no criticality to the order in which the ingredients of the final mixture are blended together, the magnesium stearate and the polytetrafluoroethylene were blended in last. Upon addition of all the ingredients the mixture was blended for about 1 to about 3 minutes. Blending for more than three minutes is not advantageous because the hardness of the final tablet will be lessened by increased blending times. The mixture was then compressed into tablets using a tablet press having a die diameter of 27/32." Each tablet weighed about 3.0 grams and had a hardness of about 16 SCU on edge Heuberlein. The tablets were cured in a hot air oven for about 60 minutes at about 90° C. The tablets were about 0.2 inches thick and upon testing were found to have available oxygen of about 115 mg/tablet with a disintegration time of maximum about 180 seconds in about 120 milliliters of 45° C (initial temperature) water. When dissolved in about 120 milliliters of water the composition yielded a pH of about 8.6.

EXAMPLE 5

Tablets made from the denture cleansing composition of Example 4 were compared, in terms of cleaning ability, to tablets made from the comparative composition presented in Table III.

## TABLE III

### Comparative Denture Cleansing Composition

| Ingredients | Amount (mg/tablet) |
|---|---|
| Sodium Bicarbonate USP Granular No. 5 | 427.2 |
| Citric Acid Anhydrous USP Fine Granular | 316.8 |
| Sodium Carbonate (Soda Ash dense) | 395.1 |
| Dyes | 2.72 |
| Water, potable | 10.00 |
| Potassium Monopersulfate Compound | 1,209.6 |
| EDTA Tetrasodium Salt dihydrate/Pure | 38.40 |
| Lathanol LAL-79 Medium Flake or Powder | 20.0 |
| Flavor | 31.0 |
| Sodium Benzoate | 48.0 |
| Magnesium Stearate | 6.72 |
| Sodium Perborate Monohydrate | 384.0 |
| Sodium Sulfate Anhydrous | 187.2 |
| Total | 3,076.74 |

The ingredients of Table III were blended together and the resulting mixture was compressed into tablets using a tablet press having a die diameter of 27/32″. Each tablet had a hardness of about 16 SCU on edge Heuberlein. The tablets were cured in a hot air oven for about 60 minutes at about 90°C. The tablets were about 0.2 inches thick and upon testing were found to have available oxygen of about 115 mg/tablet with a distintegration time of maxium about 180 seconds in about 120 milliliters of 45°C (initial temperature) water. When dissolved in water the comparative composition yielded a pH of about 8.6.

The inventive composition of Table II was compared to the Table III comparative composition by testing their abilities to clean a composite food and plaque stain from denture tiles in vitro. Denture tiles having coffee, tea, blueberry and grape stains on a plaque matrix were soaked for about 12 minutes in a solution of the Table II inventive composition made by dissolving about a 3.0 gram tablet in about 120 milliliters of water at an initial temperature of about 45°C. Similarly stained denture tiles were soaked for about 12 minutes in a solution of the Table III comparative composition made by dissolving about a 3.0 gram tablet in about 120 milliliters of water at an initial temperature of about 45°C.

The inventive composition of Table II demonstrated improved cleaning in comparison to the non-inventive composition of Table III.

This invention may be embodied in other forms or carried in other ways without departing from the spirit or essential characteristics thereof. The present disclosure is therefore to be considered as in all respects illustrative and not restrictive, the scope of the invention being indicated by the appended claims, and all changes which come within the meaning and range of equivalency are intended to be embraced therein.

## Claims

1. Effervescent bleach activator granules for use in washing, cleansing and/or bleaching compositions comprising:
   (i) a bleach activator;
   (ii) an anhydrous alkali metal perborate; and/or
   (iii) a perborate monohydrate of a metal selected from the group consisting of an alkali metal, an alkaline earth metal and mixtures thereof; and
   (iv) a polymeric fluorocarbon binder which is inert to the bleach activator.

2. The bleach activator granules according to Claim 1 wherein said anhydrous alkali metal perborate is anhydrous sodium perborate.

3. The bleach activator granules according to Claim 1 wherein said perborate monohydrate is sodium perborate monohydrate.

4. The bleach activator granules according to Claim 1 comprising from about 5 to about 50% by weight, based on the weight of the granule, of said anhydrous perborate.

5. The bleach activator granules according to Claim 1 comprising from about 10 to about 50% by weight of said bleach activator, from about 5 to about 50% by weight of said anhydrous perborate, from about 30 to about 60% by weight of said perborate monohydrate and from about 0.01 to about 1% by weight polymeric fluorocarbon, all weight percents based on the total weight of the granule.

6. The bleach activator granules according to Claim 1, wherein said bleach activator is N,N,N′, N′-tetraacetylethylenediamine.

7. The bleach activator granules according to Claim 1 having a particle size in the range of from about 10 mesh to about 100 mesh, preferably from about 12 to about 30 mesh.

8. The bleach activator granules according to Claim 1 which when in aqueous medium provide a pH within the range of from about 10 to about 11.5.

9. The bleach activator granules according to Claim 1 wherein the binder is polytetrafluoroethylene.

10. An improved effervescent cleansing composition comprising:
    (i) from about 20 to about 60% by weight, of the final composition, of a monopersulfate salt;
    (ii) a bleach activator;
    (iii) an anhydrous alkali metal perborate; and/or
    (iv) a perborate monohydrate of a metal selected from the group consisting of an alkali metal, an alkaline earth metal and mixtures thereof; and
    (v) a polymeric fluorocarbon binder which is inert to the bleach activator,wherein said bleach activator and at least a portion of said anhydrous perborate and/or perborate monohydrate and said polymeric fluorocarbon binder are present in a compacted granule mixture as claimed in anyone of the claims 1 to 9.

11. The composition of Claim 10 wherein said polymeric fluorocarbon is polytetrafluoroethylene.

12. The composition of Claim 10 wherein said monopersulfate salt comprises sodium monopersulfate.

13. A process for preparing an effervescent bleach activator granule as claimed in anyone of the claims 1 to 11 said process compring admixing an alkali metal perborate monohydrate and/or an anhydrous alkali or alkaline earth perborate and a polymeric fluorocarbon with a bleach activator in the dry state for from about 1 to about 3 minutes and compressing the resultant admixture.

14. An improved effervescent cleansing composition comprising:
    (i) from about 20% to about 60% by weight, of the total composition, of a monopersulfate salt;

(ii) an effective amount of a bleach activator;

(iii) an effective amount of an anhydrous alkali metal perborate; and/or

(iv) an effective amount of perborate monohydrate of a metal selected from the group consisting of an alkali metal, an alkaline earth metal and mixtures thereof; and

(v) an effective amount of a polymeric fluorocarbon binder which is inert to the bleach activator; wherein at least a portion of said anhydrous perborate and/or perborate monohydrate and said polymeric fluorocarbon binder are present in a compacted granulated mixture.

15. The composition of Claim 14 wherein said bleach activator is N,N,N',N'-tetraacetylethylenediamine.

16. The composition of Claim 14 wherein said bleach activator is present in amounts of about 5% to about 15% by weight of the total composition.

17. The composition of Claim 14 wherein said granulated mixture comprises anhydrous perborate and perborate monohydrate.

18. The composition of Claim 17 wherein said anhydrous perborate and perborate monohydrate are in a ratio within the range of about 1.5:1 to about 1:1.5.

19. The composition of Claim 17 wherein said anhydrous perborate is present in amounts within the range of about 2% to about 8% by weight of the total composition and said perborate monohydrate is present within the range of about 2% to about 8% by weight of the total composition.

20. An improved effervescent cleansing composition comprising:

(i) from about 20% to about 60% by weight of the total composition of a monopersulfate salt;

(ii) from about 5% to about 15% by weight of the total composition of a bleach activator;

(iii) an anhydrous alkali metal perborate and a perborate monohydrate selected from the group consisting of alkali metal perborate monohydrates, alkaline earth metal perborate monohydrates, and mixtures thereof, wherein from about 2% to about 8% by weight of the total composition of anhydrous alkali metal perborate and about 2% to about 8% by weight of the total composition of perborate monohydrate are present in a compacted granulated mixture with about 0.4% to about 0.7% by weight of the granulated mixture of a polymeric fluorocarbon, and wherein the ratio of said anhydrous perborate to said perborate monohydrate in said compacted granulated mixture is within the range of about 1.5:1 to about 1:1.5; and

(iv) an effective amount of perborate monohydrate not present within said compacted granulated mixture, said perborate monohydrate being selected from the group consisting of alkali metal perborate monohydrates, alkaline earth metal perborate monohydrates, and mixtures thereof.

21. The composition of Claims 14 or 20 wherein there is present about 7% to about 11% by weight of the total composition of perborate monohydrate not present within the compacted granulated mixture.

22. The composition of Claim 14 or 20 wherein said perborate monohydrate is an alkali metal perborate monohydrate.

23. The composition of Claim 22 wherein the alkali metal of the anhydrous alkali metal perborate and the alkali metal perborate monohydrate is sodium.

24. The composition of Claims 14 or 20 wherein said monopersulfate salt is potassium monopersulfate.

25. The composition of Claim 20 wherein said monopersulfate is potassium monopersulfate, said perborates are anhydrous sodium perborate and sodium perborate monohydrate, and said monohydrate perborate not present within said compacted granulated mixture is present in said composition in an amount of about 7% to about 11% by weight of the total composition.

26. The composition of Claim 25 further comprising effective amounts of a detergent and a chelant.

27. The composition of Claims 14 or 20 further comprising effective amounts of a detergent and/or a chelant.